# EUROPEAN PATENT APPLICATION

(11) **EP 4 144 407 A1**
(43) Date of publication of application: **08.03.2023**
(21) Application number: 21194249.5
(22) Date of filing: 01.09.2021
(51) Int. Cl.: A61N 1/362, A61N 1/37, A61B 5/11, A61B 5/00, A61N 1/05, A61N 1/372, A61N 1/36

(54) **A METHOD AND AN ARRANGEMENT FOR CONFIGURING AN IMPLANTABLE MEDICAL DEVICE FOR STIMULATING A HUMAN OR ANIMAL HEART**

(71) Applicant: BIOTRONIK SE & Co. KG, 12359 Berlin (DE)
(72) Inventor: WENZEL, Matthias, 10243 Berlin (DE)
(74) Representative: Biotronik Corporate Services SE

(57) **Abstract**

A method for configuring an implantable medical device for stimulating a human or animal heart is disclosed. The method comprising the following steps: a) stimulating (200), with a programming device (100) or an implantable medical device (102) operated under a test stimulation configuration, the heart (104) of a patient (105); b) capturing (201), with a plurality of electrode patches (108) applied onto a body surface of the patient (105), first signals being representative for an electric cardiac excitation propagation in the patient's heart (104) in response to the stimulation (200); c) capturing (202), with at least one of the plurality of electrode patches (108) applied onto the body surface of the patient (105), a second signal being representative for an excitation of the diaphragm of the patient (105) and/or for a phrenic stimulation threshold; d) determining (203), on the basis of the first signals, at least one first parameter chosen from the group consisting of a cardiac stimulation threshold, a cardiac stimulation impedance, a cardiac stroke volume, a cardiac signal amplitude, an atrioventricular interval, and an interventricular interval; e) determining (204), on the basis of the second signal, a phrenic stimulation threshold; f) optionally repeating (205) steps a) to e); g) setting (206), on the basis of the at least one first parameter and of the phrenic stimulation threshold, an operating stimulation configuration of the implantable medical device (102) that enables sufficient stimulation of the patient's heart (104) by the implantable medical device (102) and avoids at the same time a stimulation of the patient's diaphragm upon a cardiac stimulation by the implantable medical device (102).

## Description

Aspects of the present invention generally relate to a method and an arrangement for configuring an implantable medical device for stimulating a human or animal heart. The implantable medical device to be configured serves for providing an intra-cardiac pacing function, in particular a ventricular pacing.

Implantable medical devices for stimulating a human or animal heart, such as pacemakers, have been known for a long time. They can perform different functions. Different stimulation programs can be carried out by an appropriate pacemaker to restore the treated heart to a normal state.

The choice of an appropriate stimulation program (often also referred to as stimulation vector or stimulation configuration) is still a demanding task. According to prior art solutions, the stimulation vector is chosen based on an automatically measured stimulation threshold and a cardiac impedance. In addition, solutions are known in which (undesired) stimulations of the diaphragm of the patient are manually measured in a particularly time-consuming manner.

Furthermore, it should be noted that all physiologic parameters, in particular a cardiac stimulation threshold and a phrenic stimulation threshold, will typically change postoperatively.

Known stimulation strategies including an optimization of the atrioventricular interval and the interventricular interval based on an electrocardiogram (ECG) are typically not sufficient. This is due to the fact that they do not allow a non-invasive control of success during the implantation. To be more precise, the ECG results are typically inaccurate. Furthermore, an additional application of an echocardiography is too complex for standard implantation procedures.

In some instances, the intracardiac impedance is used as a surrogate for the cardiac stroke volume. However, this impedance is not specific enough since complex contraction movements of the heart influence a correct correlation between the impedance and the cardiac stroke volume.

Thus, the configuration of an implantable medical device for stimulating a heart still requires optimization.

It is an object of the present invention to provide the possibility of a patient-specific dynamic optimization of a cardiac stimulation.

This object is achieved with a method for configuring an implantable medical device for stimulating a human or animal heart comprising the steps explained in the following.

In a first step, a programming device or an implantable medical device operated under a test stimulation configuration is used to stimulate the heart of a patient.

In another method step, a plurality of electrode patches applied onto a body surface of the patient is used to capture first signals. These first signals are representative for an electric cardiac excitation propagation within the patient in response to the previously applied stimulation. The electrode patches do not require an invasive interaction with the patient's body. Rather, they can be simply adhered to the skin of the patient, typically on the torso of the patient. These electrode patches form, in an embodiment, part of a body surface mapping system (also referred to as BSM). The patches can also be integrated in a vest to be worn by the patient on the skin. Thus, it is not necessary to form an adhesive bond between the electrode patches and the body surface of the patient. Rather, any close interaction between the electrode patches and the body surface of the patient suffices to allow capturing the first signals.

The plurality of electrode patches comprises, in an embodiment, at least 2 electrode patches, in particular 2 to 20, in particular 3 to 19, in particular 4 to 18, in particular 5 to 17, in particular 6 to 16, in particular 7 to 15, in particular 8 to 14, in particular 9 to 13, in particular 10 to 12 electrode patches. Each electrode patch comprises at least one electrode designed and configured to detect an electric signal of the patient's heart.

At least one of the plurality of electrode patches also comprises a sensor for detecting an excitation of the diaphragm of the patient and for determining a phrenic stimulation threshold. Such a sensor can be, e.g., a motion sensor such as an acceleration sensor. This additional sensor is used to capture a second signal that is representative for an excitation of the diaphragm of the patient and/or for a stimulation threshold of the diaphragm of the patient. Thus, the electrode patches serve both for capturing electric signals being indicative of the cardiac activity of the patient as well as motion signals being indicative of a phrenic activity of the patient.

Afterwards, at least one first parameter is determined on the basis of the first signals. The at least one first parameter is chosen from the group comprising, in particular consisting of a cardiac stimulation threshold, a cardiac stimulation impedance, a cardiac stroke volume, the cardiac signal amplitude, an atrioventricular interval, and an interventricular interval. Thus, the detected first signals are translated into physiologic parameters.

In another method step, a phrenic stimulation threshold is determined on the basis of the second signal. Consequently, both cardiac parameters as well as a phrenic parameter are provided for any further decisions to be made.

It is possible to repeat the precedingly explained steps of stimulating the heart of the patient, of capturing the first signals in response to the stimulation, of capturing the second signal representative for a phrenic activity and of determining both physiologic cardiac parameters as well as the phrenic stimulation threshold once or for a plurality of times. In doing so, typically a different test stimulation configuration is chosen to stimulate the heart. Thus, it is made possible to obtain information on how a change in the test stimulation configuration affects the determined physiologic cardiac and phrenic parameters. Thus, there is always a direct link between the applied test stimulation configuration and the resulting physiologic parameters.

In a further method step, an operating stimulation configuration of the implantable medical device is set on the basis of the at least one first parameter and on the basis of the phrenic stimulation threshold. This operating simulation configuration (operating stimulation vector) ensures a sufficiently high stimulation of the patient's heart by the implantable medical device and avoids at the same time a stimulation of the patient's diaphragm upon a cardiac stimulation by the implantable medical device. A sufficient stimulation or a sufficiently high stimulation is achieved when the applied stimulation is able to return the stimulated heart back to its physiologic function.

Due to the detection of signals being representative for phrenic function together with the detection of signals being indicative for cardiac function, the presently described method allows a particular appropriate configuration of the implantable medical device so that a stimulation applied by this medical device can be performed under particular physiologic conditions.

The presently described method employs a three-dimensional parametrization of the electric excitation propagation within the patient's heart. It enables a reconstruction of the volumes of all cardiac chambers in real time over a plurality of cardiac cycles. This enables a determination of contraction movements of the left ventricle and its stroke volume in dependence on the contraction movement. An intracardiac impedance measurement between a right ventricular electrode and each pole of a multipolar (e.g., quadrupolar) left ventricular electrode can thus be corrected by the contraction movements of the left ventricle. Thus, a correlation between the intracardiac impedance and the left ventricular stroke volume is made possible. The corresponding correlation factor as well as the resulting measuring results can be stored in the implantable medical device as reference parameter (basic reference). It is also possible to transfer these values to a home monitoring service center (cf. below for further details). The impedance measurement can be regularly repeated by measuring the impedance between the right ventricular electrode and the chosen poles of the multipolar left ventricular electrode. Then, the stroke volume of the left ventricle can be regularly determined. The left ventricular stroke volume can be used as indicator of a therapy success of the cardiac stimulation applied by the implantable medical device.

In an embodiment, the operating stimulation configuration to be set is automatically chosen based on the at least one first parameter and the phrenic stimulation threshold. Then, no user interaction is necessary for setting the operating stimulation configuration so that the methods can be carried out in a particularly simple manner.

In an alternative embodiment, appropriate parameters, in particular the optimum parameters, for a specific stimulation configuration are indicated to a user, wherein the user is then allowed to set the stimulation configuration on the basis of these parameters. This embodiment allows a tighter control of the parameters of the operating stimulation configuration to be set by a user, in particular by medical staff. Depending on regulatory requirements, it might be necessary or recommended to apply this embodiment.

In an embodiment, the method further comprises a step of reconstructing a three-dimensional model of the heart of the patient. This model visualizes a time-dependent electric cardiac excitation propagation on the basis of the captured first signals. The three-dimensional model can have different appearances. To give an example, it is possible to visualize the electric cardiac excitation propagation in different colors so as to allow an easy comprehension of the excitation propagation for a human user. Furthermore, the three-dimensional model can be displayed together with the parameters used for calculating the model, i.e., together with one or more first parameters. In addition, it is also possible to display the three-dimensional model together with the calculated phrenic stimulation threshold or an indicator whether or not phrenic stimulation has been taken place when applying a specific stimulation configuration. This will allow a user a particularly easy overview on the physiologic conditions of the stimulated heart and the diaphragm of the patient under different stimulation configurations. Then, the choice of a particular appropriate operating stimulation configuration is further facilitated.

In an embodiment, the three-dimensional model of the heart comprises information on a volume of at least one of the right atrium, the right ventricle, the left atrium, and the left ventricle of the heart to be stimulated. This three-dimensional volume information delivers particularly valuable physiologic information on the cardiac state of the heart to be stimulated. In contrast to using the intracardiac impedance as surrogate for the cardiac stroke volume, the calculation of three-dimensional information on the volumes of the individual heart chambers allows a particular accurate and reliable calculation of the cardiac stroke volume. Since the cardiac stroke volume is one of the most important physiologic cardiac parameters to evaluate the proper functioning of the heart, such reliable volume information is of particular medical impact. Reliable three-dimensional information can be particularly easy obtained if the electrode patches are applied onto the front side and onto a backside of the patient's body surface, in particular of the patient's torso.

In an embodiment, the at least one first parameter is the cardiac stroke volume since it has a high medical impact, as outlined above.

In an embodiment, the step of determining at least one first parameter comprises determining at least 2, in particular at least 3, in particular at least 4, in particular at least 5, in particular all first parameters.

In an embodiment, the operating stimulation configuration to be set is chosen based on an optimization process. For this purpose, the operating simulation configuration is chosen such that a stimulation applied in this operating stimulating configuration will result in the lowest possible cardiac stimulation threshold necessary and sufficient to obtain the highest possible cardiac stroke volume without stimulating the patient's diaphragm. The lowest possible cardiac stimulation threshold will enable a particularly physiologic stimulation of the heart without applying too intense stimulation pulses. If the highest possible cardiac stroke volume is achieved, a particularly physiologic function of the stimulated heart is given. These two parameters are chosen such that an undesired and often painful stimulation of the patient's diaphragm is avoided. The parameters to be chosen according to this embodiment can be visualized in a diagram and can be generally chosen according to known optimization processes. If the lowest possible cardiac stimulation threshold with the highest possible cardiac stroke volume have been chosen will be assessed, in this embodiment, on the basis of the determined first parameters that define the pool of possible cardiac stimulation thresholds and cardiac stroke volumes.

In an embodiment, the operating stimulation configuration is chosen such that a stimulation applied under this operating stimulation configuration will result in the best possible atrioventricular conductor and the best possible interventricular activation. Once again, an assessment whether or not the best possible physiologic parameters have been chosen will be assessed on the basis of the determined first parameters. Thus, the term "best" used in this and in the precedingly explained embodiment do not refer to the best physiologic condition in absolute terms, but rather in relative terms with respect to the pool of the determined first parameters in which the best parameters are chosen in these embodiments.

In an embodiment, the method further comprises the step of storing at least one first parameter resulting from a cardiac stimulation under the operating stimulation configuration as reference parameter (basic reference) in a memory unit of the implantable medical device. It is also possible to transfer the stored first parameter to the programming device and from there to a home monitoring service center to be able to restore the stored first parameter at a later time point from any of these devices, if desired. Storing at least one of the first parameters as reference parameters will enable a user to perform a follow-up of the efficacy of the chosen operating stimulation configuration. Then, a trend analysis of the physiologic response to the applied cardiac stimulation is made possible.

In an embodiment, the method comprises such follow-up step. For this purpose, at least one first parameter resulting from a cardiac stimulation under the chosen operating stimulation configuration is regularly determined and compared with the same parameter stored as reference parameter in the memory unit of the implantable medical device. Any observed deviations can be used to determine a time-dependent change of the efficacy of the chosen operating stimulation configuration. It is also possible to store the first parameter regularly determined after determining the reference parameter in the implantable medical device. Furthermore, it is also possible to transfer this later determined first parameter to the programming device and/or to a home monitoring service center, if desired. The trend of the change in the physiologic parameters resulting from the applied operating stimulation configuration can then be used to amend the operating stimulation configuration at a later time point. Thus, the method enables, in this embodiment, a dynamic readjustment of the chosen operating stimulation configuration and thus a particularly appropriate long-term physiologic stimulation of the patient's heart. In doing so, it is also particularly easy to compensate for post-operative changes of physiologic responses to applied cardiac stimulations. Consequently, the presently described method serves for a long-term physiologic stimulation of the patient's heart due to regular readjustment of the operating stimulation configuration in dependence on determined physiologic parameters indicating, on the one hand, a cardiac stimulation effect and, on the other hand, an avoidance of a phrenic stimulation due to applied stimulating or pacing pulses by the implantable medical device.

In an aspect, the present invention relates to a method for implanting an implantable medical device for stimulating a human or animal heart. This method comprises the steps explained the following.

First, a plurality of electrode patches is applied onto a body surface of the front and back torso of the patient. These electrode patches can be directly applied onto the skin of the patient, e.g., by adhesive forces. Alternatively, the patches can be integrated into a vest to be worn by a patient being thus in close interaction with the body surface of the torso of the patient.

Afterwards, the plurality of electrode patches is used for capturing first electric signals during a plurality of cardiac cycles of the patient. These first signals are representative for an electric cardiac excitation propagation within the patient's heart.

In a further method step, at least one first parameter is determined on the basis of the first signals. This first parameter is chosen from the group consisting of a cardiac stimulation threshold, a cardiac stimulation impedance, a cardiac stroke volume, a cardiac signal amplitude, an atrioventricular interval, an intraventricular excitation interval, and in interventricular interval.

In a further method step, a three-dimensional model of the heart of the patient is reconstructed on the basis of the captured first signals. This three-dimensional model reflects the time-dependent electric cardiac excitation propagation of the patient's heart. The determined first parameters can also be displayed together with the reconstructed three-dimensional model of the heart.

The reconstructed three-dimensional model of the heart then serves for visually determining an appropriate position of a left ventricular electrode to be implanted.

Finally, intracardiac stimulation electrodes of an implantable medical device for stimulating the patient's heart are positioned and implanted. To be more precise, the intracardiac stimulation electrodes are implanted in the right ventricle (right ventricular electrode), in the right atrium (right atrial electrode) and within a coronary nervous system above the left ventricle (left ventricular electrode).

Summarizing, this implantation method makes use of information gathered by electrode patches applied onto the patient's body surface in order to be able to implant the electrodes of the implantable medical device in a physiologic particularly sensible way.

In an embodiment, the implantation method further comprises the steps explained in the following. The steps succeed the step of positioning and implanting the intracardiac stimulation electrodes and serve for configuring the implanted medical device in a particular appropriate way to achieve a highly physiologic stimulation of the heart to be stimulated.

In a first step, a programming device or the already implanted implantable medical device operated under a test stimulation configuration is used to stimulate the heart of the patient.

In another method step, the plurality of electrode patches applied onto the body surface of the patient is used to capture third signals. These third signals are representative for an electric cardiac excitation propagation within the patient's heart in response to the previously applied stimulation.

Each electrode patch comprises at least one electrode designed and configured to detect an electric signal of the patient's heart. At least one of the plurality of electrode patches also comprises a sensor for detecting an excitation of the diaphragm of the patient and for determining a phrenic stimulation threshold. Such a sensor can be, e.g., a motion sensor such as an acceleration sensor. This additional sensor is used to capture a second signal that is representative for an excitation of the diaphragm of the patient and/or for a stimulation threshold of the diaphragm of the patient. Thus, the electrode patches serve both for capturing electric signals being indicative of the cardiac activity of the patient as well as motion signals being indicative of a phrenic activity of the patient.

Afterwards, at least one third parameter is determined on the basis of the third signals. The at least one third parameter is chosen from the group comprising, in particular consisting of, a cardiac stimulation threshold, a cardiac stimulation impedance, a cardiac stroke volume, the cardiac signal amplitude, an atrioventricular interval, and an interventricular interval. Thus, the detected first signals are translated into physiologic parameters.

In another method step, a phrenic stimulation threshold is determined on the basis of the second signal.

It is possible to repeat the precedingly explained steps of stimulating the heart of the patient, of capturing the third signals in response to the stimulation, of capturing the second signal representative for a phrenic activity and of determining both physiologic cardiac parameters as well as the phrenic stimulation threshold once or for a plurality of times. In doing so, typically a different test stimulation configuration is chosen to stimulate the heart. Thus, it is made possible to obtain information on how a change in the test stimulation configuration affects the determined physiologic cardiac and phrenic parameters.

In a further method step, an operating stimulation configuration of the implantable medical device is set on the basis of the at least one third parameter and on the basis of the phrenic stimulation threshold. This operating simulation configuration ensures a sufficiently high stimulation of the patient's heart by the implantable medical device and avoids at the same time a stimulation of the patient's diaphragm upon a cardiac stimulation by the implantable medical device.

The parameters referred to as "third parameters" in the precedingly explained embodiment are generally the same parameters as the "first parameters" explained with respect to previous embodiments. Thus, all explanations given with respect to the first parameters in the preceding embodiments can be directly transferred to the third parameters of the present and the succeeding embodiments.

In an embodiment, at least one first parameter (resulting from a physiologic cardiac cycle) and at least one third parameter (resulting from a cardiac stimulation under the operating stimulation configuration) are stored as reference parameters in a memory unit of the implantable medical device. In doing so, both the physiologic conditions prior to the implantation of the implantable medical device as well as after its implantation and optimization of the stimulation configuration are stored and can be subsequently used in a follow-up to evaluate any change in the physiologic reaction of the patient to a stimulation applied by the implantable medical device.

In a further embodiment, at least one further parameter resulting from a cardiac stimulation under the operating stimulation configuration is regularly determined and compared with the same third parameter or the same first parameter stored as reference parameter in the memory unit of the implantable medical device. Based on such a comparison, a follow-up of the physiologic effects of a stimulation applied by the implantable medical device can be particularly easy carried out.

In an aspect, the present invention relates to an arrangement for configuring the implantable medical device for stimulating a human or animal heart. This arrangement comprises an implantable medical device for stimulating a human or animal heart. It further comprises a programming device operatively coupled to the implantable medical device.

The arrangement further comprises a plurality of electrode patches. These electrode patches can be applied onto a body surface of the patient. In doing so, the electrode patches can be directly applied onto the body surface of the patient or they can be integrated into a vest that is to be worn by a patient. The plurality of electrode patches is capable of sensing first signals being representative for an electric cardiac excitation propagation within the patient's heart when the electrode patches are applied onto the body surface of the patient. At least one of the plurality of electrode patches comprises a sensor for determining a phrenic stimulation of the patient, such as a motion sensor.

The programming device comprises a processor and a memory unit. The memory unit comprises computer-readable code that causes the processor to execute a method comprising the steps explained in the following when executed on the processor.

In a first step, the programming device or the implantable medical device is operated under a test stimulation configuration and is used to stimulate the heart of the patient.

In another method step, the plurality of electrode patches after having been applied onto the body surface of the patient is used to capture first signals. These first signals are representative for an electric cardiac excitation propagation within the patient's heart in response to the previously applied stimulation.

At least one of the electrode patches is used to capture a second signal that is representative for an excitation of the diaphragm of the patient and/or for a stimulation threshold of the diaphragm of the patient. Thus, the electrode patches serve both for capturing electric signals being indicative of the cardiac activity of the patient as well as motion signals being indicative of a phrenic activity of the patient.

Afterwards, at least one first parameter is determined on the basis of the first signals. The at least one first parameter is chosen from the group comprising, in particular consisting of, a cardiac stimulation threshold, a cardiac stimulation impedance, a cardiac stroke volume, the cardiac signal amplitude, an atrioventricular interval, and an interventricular interval. Thus, the detected first signals are translated into physiologic parameters.

In another method step, a phrenic stimulation threshold is determined on the basis of the second signal.

It is possible to repeat the precedingly explained steps of stimulating the heart of the patient, of capturing the first signals in response to the stimulation, of capturing the second signal representative for a phrenic activity and of determining both physiologic cardiac parameters as well as the phrenic stimulation threshold once or for a plurality of times. In doing so, typically a different test stimulation configuration is chosen to stimulate the heart. Thus, it is made possible to obtain information on how a change in the test stimulation configuration affects the determined physiologic cardiac and phrenic parameters.

In a further method step, an operating stimulation configuration of the implantable medical device is set on the basis of the at least one first parameter and on the basis of the phrenic stimulation threshold. This operating simulation configuration ensures a sufficiently high stimulation of the patient's heart by the implantable medical device and avoids at the same time a stimulation of the patient's diaphragm upon a cardiac stimulation by the implantable medical device.

In an embodiment, the implantable medical device is an implantable pulse generator (IPG), an implantable cardioverter-defibrillator (ICD), or a device for cardiac resynchronization therapy (CRT). A CRT device is particularly appropriate.

In an embodiment, the arrangement further comprises a remotely located home monitoring service center (HMSC), wherein the programming device is operatively coupled to the home monitoring service center. Then, the programming device is able to transfer data to the home monitoring service center. Thus, the programming device serves as interface between the implantable medical device and/or the electrode patches on the one hand and the HMSC on the other hand. Such a configuration allows a particularly easy centrally managed monitoring of the implantable medical device and its functioning over an extended period of time.

All embodiments of the explained methods can be combined in any desired way and can be transferred either individually or in any arbitrary combination to the respective other method or to the arrangement. Likewise, all embodiments explained with respect to the arrangement can be combined in any desired way and can be transferred either individually or in any arbitrary combination to any of the described methods.

Further details of aspects of the present invention will be described with respect to exemplary embodiments and accompanying Figures. In the Figures:
- Figure 1: schematically shows the structure of an embodiment of an arrangement for configuring an implantable medical device for stimulating a human or animal heart; and
- Figure 2: schematically illustrates individual steps of an embodiment of a method for configuring an implantable medical device for stimulating a human or animal heart.

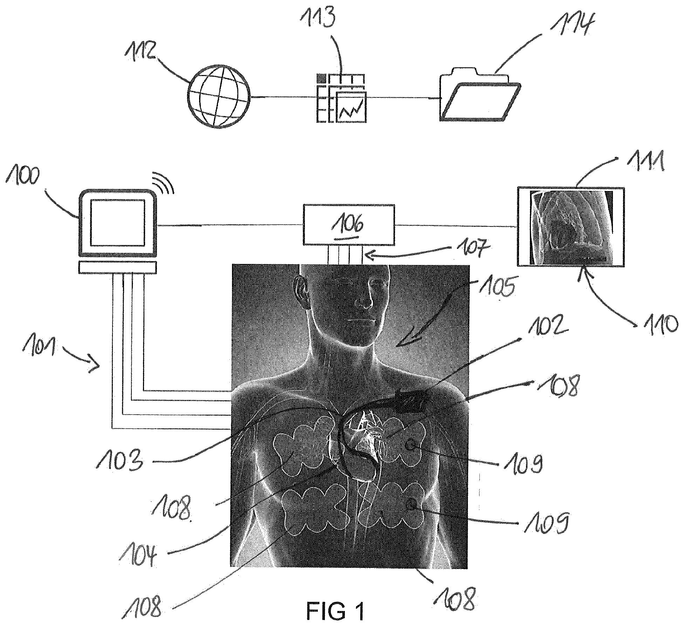

Figure 1 is a schematic depiction of an embodiment of an arrangement for configuring an implantable medical device. This arrangement comprises a programmer 100 serving as programming device. The programmer 100 comprises a plurality of first measuring connections 101 serving to connect the programmer 100 with an implantable medical device 102. The implantable medical device 102 comprises intracardiac electrodes 103, one of which is implanted into the right ventricle of a heart 104 of a patient 105, and one of which is implanted within the coronary nervous system above the left ventricle of the heart 104.

The arrangement further comprises a measuring station 106 being operatively connected to the programmer 100. In other implementations, the measuring station 106 forms an integral part of the programmer 100. It comprises a plurality of second measuring connections 107 serving for connecting the measuring station 106 with a plurality of electrode patches 108 directly applied onto the skin of the patient 105. Two of the electrode patches 108 do not only comprise electrodes, but also comprise a motion sensor 109 by which the detection of the phrenic activity of the patient 105 is possible. The data gathered by the measuring station 106 is transferred to the programmer 100. Thus, the programmer 100 is in receipt of cardiac electric signals obtained via the implantable medical device 102 as well as by the electrode patches 108. Furthermore, the programmer 100 is in receipt of signals indicating the phrenic activity of the patient 105 based on the measurements of the motion sensors 109.

Based on this data, the programmer 100 is able to reconstruct a 3-D model 110 of the heart 104 of the patient 105 that can be displayed on a display 111 being connected to the programmer 100. This reconstructed three-dimensional model 110 of the heart 104 illustrates the electric cardiac excitation propagation in the time-dependent manner to illustrate the cardiac function to a user of the arrangement.

By determining physiologic parameters in response to a cardiac stimulation by the implantable medical device 102, the programmer 100 can determine the physiologic effect of the performed stimulation. By applying different test stimulation configurations of the implantable medical device 102, the different physiologic effects can be recorded. Subsequently, the programmer 100 is able to indicate a user the best possible stimulation configuration. The user can then individually set this stimulation configuration in the implantable medical device 102. It is also possible that the programmer 100 automatically sets the optimum determined stimulation configuration of the implantable medical device 102.

The data gathered by the programmer 100 is then transferred via a global network 112, such as the Internet, to a home monitoring service center 113. Thus, it is made possible to remotely configure the implantable medical device 102 from the home monitoring service center 113 as well as to monitor the proper functioning of the implantable medical device 102 from there over an extended period of time. Furthermore, it is made possible to compare currently obtained data on the physiologic effect of the cardiac stimulation applied by the implantable medical device 102 with earlier obtained and stored data. In doing so, it is possible to conduct a follow-up analysis of the functioning of the implantable medical device 102. Furthermore, a readjustment of the stimulation configuration settings of the implantable medical device 102 is thus made particularly easy.

The home monitoring service center 113 can be connected to an electronic health record 114 to store relevant patient-related data in the electronic health record 114.

Summarizing, the arrangement depicted in Figure 1 presents an easy way to allow an initial configuration setting of the implantable medical device 102 as well as a continuous or regular monitoring of the functioning of the implantable medical device 102 via a remote access from the health monitoring service center 113 via the programmer 100. Due to the visualization of the reconstructed model 110 of the heart 104, the physiologic effects of the stimulation applied by the implantable medical device 102 are made particularly easy available for human use. By also monitoring the phrenic activity of the patient 105 by motion sensors 109, the stimulation configuration of the implantable medical device 102 is set such that a cardiac stimulation will not result in a phrenic stimulation. This increases the comfort of any stimulation applied by the implantable medical device 102 and reduces side effects of such stimulation.

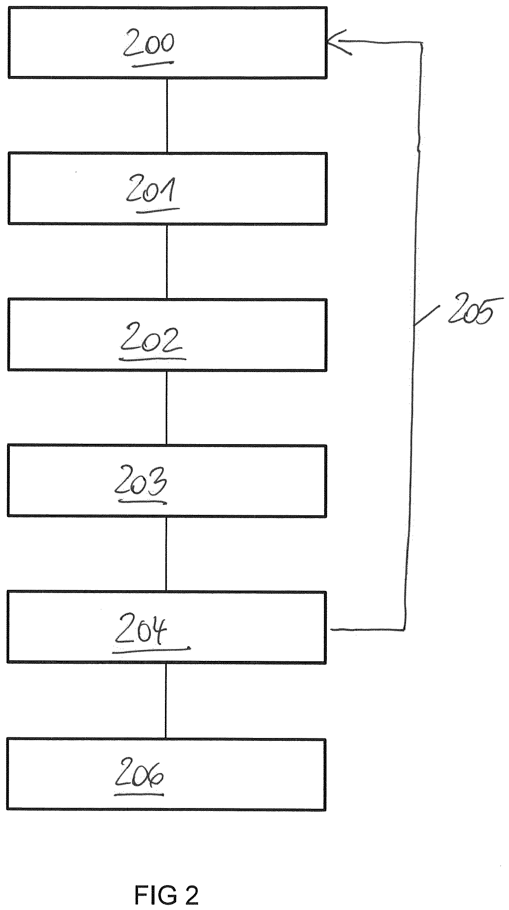

Figure 2 schematically illustrates a process of an embodiment of a method for configuring an implantable medical device for stimulating the heart.

In a first step 200, a programming device or an implantable medical device is used to stimulate the heart of the patient. For this purpose, the programming device or the implantable medical device is operated under a test stimulation configuration. Afterwards, electric signals being representative for a cardiac excitation propagation within the patient's heart are captured in a second step 201. This is done with a plurality of electrode patches applied onto a body surface of the patient.

In a third step 202, a second signal being representative for an excitation of the diaphragm of the patient and/or for a phrenic stimulation threshold is captured. This is done with at least one of the plurality of electrode patches applied onto the body surface of the patient. For this purpose, this electrode patch comprises a sensor being appropriate for detecting phrenic signals, such as a motion sensor. The third step 202 can be performed prior to or concomitantly with the second step 201. Typically, the cardiac signals and the phrenic signals are sensed at the same time.

In a forth step 203, a cardiac parameter is determined on the basis of the previously captured first signals. This cardiac parameter is chosen from the group comprising a cardiac stimulation threshold, a cardiac stimulation impedance, a cardiac stroke volume, a cardiac signal amplitude, an atrioventricular interval, and an interventricular interval.

In a fifth step 204, a phrenic stimulation threshold is determined on the basis of the second signal. The fifth step 204 can be done prior to or concomitantly with the fourth step 203. Typically, the first cardiac parameter and the phrenic stimulation threshold are determined at the same time.

All steps beginning from the first steps 200 to the fifth step 204 can now be repeated in a repeating loop 205 if desired. In doing so, typically a different test stimulation configuration of the programming device or of the implantable medical device is applied to obtain additional information on the behavior of the body of the patient in response to a cardiac stimulation performed under different test stimulation configurations. Thus, by repeating the first step 200 to the fifth step 204, different first cardiac parameters and different phrenic stimulation thresholds can be obtained. Thus, different physiologic effects of different test stimulation configurations are established.

In a concluding step 206, an operating stimulation configuration of the implantable medical device is set on the basis of the at least one first parameter and of the phrenic stimulation threshold previously determined. This operating stimulation configuration will enable a sufficient stimulation of the patient's heart by the implantable medical device. At the same time, it avoids a stimulation of the patient diaphragm upon a cardiac stimulation by the implantable medical device. Thus, the process illustrated in Figure 2 ensures the choice of an operating stimulation configuration of the implantable medical device that ensures a physiologic relevant cardiac stimulation but avoids an undesired phrenic stimulation.

The method explained in Figure 2 can form part of a more complex implantation method that will be explained in the following in detail.

For carrying out this implantation method, body surface mapping patches are applied onto the front and back torso of a patient. Likewise, the patient can be provided with a vest comprising electrodes electrode patches. These body surface mapping patches form a body surface mapping system.

The body surface mapping system is then connected with a programmer and with an external display. The electric excitation propagation in the patient's heart is then sensed over a plurality of cardiac cycles by the body surface mapping system. This includes gathering information on an atrioventricular interval, on an interventricular interval and on intraventricular excitation times.

Based on the obtained data, a three-dimensional model of the patient's heart is reconstructed. This model includes information on the electric excitation propagation and is visualized on the display. This visualization includes a colored representation of the course of the excitation propagation. The three-dimensional model also visualizes the atrioventricular interval, the interventricular interval and the cardiac contraction dynamics over time.

The reconstructed model of the heart serves then for visually determining an optimal position of a left ventricular electrode along a distinct anatomic and electric landmark. For this purpose, the availability of an appropriate target vein, a distance to a right ventricular electrode to be applied at a later stage, an electrically active region and a region of the latest possible electric activation are considered.

Afterwards, the intracardiac stimulation electrodes are positioned and implanted in the right ventricle, the right atrium, and within the current nervous system above the left ventricle.

The implanted intracardiac stimulation electrodes are then connected to the programmer. The programmer then automatically measures the cardiac stimulation threshold, the stimulation impedance, and the measuring amplitude for the possible stimulation vectors of all electrodes.

At the same time, a possible stimulation of the diaphragm of the patient is captured. In doing so, the phrenic stimulation threshold is determined for all stimulation vectors of the left ventricular electrode. For this purpose, at least one sensor in the body surface mapping system is used.

Afterwards, appropriate stimulation vectors for stimulating the left ventricle are chosen based on the previously determined parameters. This is done in dependency on an optimum atrioventricular interval and an optimum interventricular activation.

The resulting excitation propagation and contraction dynamics is determined with the body surface mapping system for all pre-chosen stimulation vectors for stimulating the left ventricle. The stimulation is carried out as simple stimulation or as multiple stimulation within a cardiac cycle using a quadrupolar or multipolar electrode. The determined excitation propagation is visualized on an external display.

The optimum values for the atrioventricular interval, the interventricular interval and the intraventricular excitation propagation (in case of multiple stimulation with a quadrupolar or multipolar electrode) are determined in dependence on the resulting cardiac stroke volume of the left ventricle. This stroke volume of the left ventricle is determined by the three-dimensional reconstruction of the beating heart over one or more cardiac cycles. The determined parameters can then be visualized either as digits or in form of information in the reconstructed three-dimensional model of the heart.

The determined cardiac stroke volume is compared with the intracardiac impedance measured between the right ventricular electrode and at least one pole of the left ventricular electrode.

Afterwards, measuring vectors between the right ventricular electrode and all poles of the left ventricular electrodes are determined in reference to other measuring vectors (e.g., between the right ventricular electrode and the right atrial electrode, or between the individual poles of the left ventricular electrode and the right atrial electrode). The optimum measuring vector between the right ventricular electrode and all poles of the left ventricular electrode are also determined. These measuring vectors are then used to determine the intracardiac impedance being representative for the cardiac stroke volume of the left ventricle. All determined parameters are stored in the programmer.

Afterwards, the intracardiac electrodes that have been still connected to the programmer are connected to the implantable medical device, in particular to a device for cardiac re-synchronisation therapy (CRT device).

Subsequently, all measurements that have already been done are repeated with the implantable medical device. All parameters that have been determined prior to the implantation of the implantable medical device and that have been determined after optimizing the stimulation configuration of the implantable medical device are then stored as basic reference in the implantable medical device. Furthermore, the resulting stimulation program (stimulation configuration) is transferred to the implantable medical device.

In addition, all parameters determined prior to the implantation of the implantable medical device and after the optimization process are telemetrically transferred to a home monitoring service center and are stored as basic references in the home monitoring service center.

At that point, the implantation procedure is terminated.

As a follow-up, all stimulation and measuring parameters are regularly (or continuously) detected and compared with the previously stored basic reference values. The result is transferred to the home monitoring service center. This allows identifying any systematic change in the physiologic response to a specific stimulation and further allows a readjustment of the stimulation configuration of the implantable medical device at a later stage, e.g., months or years after the implantation of the implantable medical device. Consequently, the previously explained implantation method employing the also previously explained configuration method ensures a stable, reliable and physiologically relevant function of the implantable medical device over its whole lifetime.

## Claims

1. A method for configuring an implantable medical device for stimulating a human or animal heart, the method comprising the following steps:
a) stimulating (200), with a programming device (100) or an implantable medical device (102) operated under a test stimulation configuration, the heart (104) of a patient (105);
b) capturing (201), with a plurality of electrode patches (108) applied onto a body surface of the patient (105), first signals being representative for an electric cardiac excitation propagation in the patient's heart (104) in response to the stimulation (200);
c) capturing (202), with at least one of the plurality of electrode patches (108) applied onto the body surface of the patient (105), a second signal being representative for an excitation of the diaphragm of the patient (105) and/or for a phrenic stimulation threshold;
d) determining (203), on the basis of the first signals, at least one first parameter chosen from the group consisting of a cardiac stimulation threshold, a cardiac stimulation impedance, a cardiac stroke volume, a cardiac signal amplitude, an atrioventricular interval, and an interventricular interval;
e) determining (204), on the basis of the second signal, a phrenic stimulation threshold;
f) optionally repeating (205) steps a) to e);
g) setting (206), on the basis of the at least one first parameter and of the phrenic stimulation threshold, an operating stimulation configuration of the implantable medical device (102) that enables sufficient stimulation of the patient's heart (104) by the implantable medical device (102) and avoids at the same time a stimulation of the patient's diaphragm upon a cardiac stimulation by the implantable medical device (102).

2. The method according to any of the preceding claims, **wherein** the operating stimulation configuration to be set is automatically chosen based on the at least one first parameter and the phrenic stimulation threshold.

3. The method according to claim 1 or 2, further comprising reconstructing a three-dimensional model (110) of the heart (104) of the patient (105) reflecting a time-dependent electric cardiac excitation propagation on the basis of the captured first signals.

4. The method according to claim 3, **wherein** the three-dimensional model (110) comprises information on a volume of at least one of a right atrium, a right ventricle, a left atrium, and a left ventricle.

5. The method according to any of the preceding claims, **wherein** the at least one first parameter is the cardiac stroke volume.

6. The method according to any of the preceding claims, **wherein** the operating stimulation configuration is chosen such that a stimulation applied under this operating stimulation configuration will result, assessed on the determined first parameters, in the lowest possible cardiac stimulation threshold with the highest possible cardiac stroke volume without stimulation of the diaphragm.

7. The method according to any of the preceding claims, **wherein** the operating stimulation configuration is chosen such that a stimulation applied under this operating stimulation configuration will result, assessed on the determined first parameters, in the best possible atrioventricular conductor and the best possible interventricular activation.

8. The method according to any of the preceding claims, further comprising storing at least one first parameter resulting from a cardiac stimulation under the operating stimulation configuration as reference parameter in a memory unit of the implantable medical device (102).

9. The method according to claim 8, further comprising regularly determining at least one first parameter resulting from a cardiac stimulation under the operating stimulation configuration and comparing it with the same parameter stored as reference parameter in the memory unit of the implantable medical device (102).

10. A method for implanting an implantable medical device for stimulating a human or animal heart, the method comprising the following steps:
applying a plurality of electrode patches (108) onto a body surface of a front and back torso of a patient (105);
capturing, with the plurality of electrode patches (108), during a plurality of cardiac cycles of the patient first signals being representative for an electric cardiac excitation propagation;
determining, on the basis of the first signals at least one first parameter chosen from the group consisting of a cardiac stimulation threshold, a cardiac stimulation impedance, a cardiac stroke volume, a cardiac signal amplitude, an atrioventricular interval, an intraventricular excitation interval, and an interventricular interval;
reconstructing a three-dimensional model (110) of the heart (104) of the patient (105) reflecting a time-dependent electric cardiac excitation propagation on the basis of the captured first signals;
visually determining, on the basis of the reconstructed three-dimensional model (110) of the heart (104), an appropriate position of a left ventricular electrode; and
positioning and implanting intracardiac stimulation electrodes (103) of an implantable medical device (102) for stimulating the patient's heart (104) in a right ventricle, in a right atrium and within a coronary nervous system above a left ventricle.

11. The method according to claim 10, further comprising the following steps succeeding the step of positioning and implanting the intracardiac stimulation electrodes (103):
a) stimulating (200), with a programming device (100) or with the implantable medical device (102) operated under a test stimulation configuration, the heart (104) of the patient (105);
b) capturing (201), with the plurality of electrode patches (108), third signals being representative for an electric cardiac excitation propagation within the patient's heart (104) in response to the stimulation;
c) capturing (202), with at least one of the plurality of electrode patches (108) applied onto the body surface of the patient (105), a second signal being representative for an excitation of the diaphragm of the patient and/or for a phrenic stimulation threshold;
d) determining (203), on the basis of the third signals, at least one third parameter chosen from the group consisting of a cardiac stimulation threshold, a cardiac stimulation impedance, a cardiac stroke volume, a cardiac signal amplitude, an atrioventricular interval, and an interventricular interval;
e) determining (204), on the basis of the second signal, a phrenic stimulation threshold;
f) optionally repeating (205) steps a) to e);
g) setting (206), on the basis of the at least one third parameter and of the phrenic stimulation threshold, an operating stimulation configuration of the implantable medical device (102) that enables sufficient stimulation of the patient's heart (104) by the implantable medical device (102) and avoids at the same time a stimulation of the patient's diaphragm upon a cardiac stimulation by the implantable medical device (102).

12. The method according to claims 10 and 11, further comprising storing at least one first parameter and at least one third parameter resulting from a cardiac stimulation under the operating stimulation configuration as reference parameter in a memory unit of the implantable medical device (102).

13. The method according to claim 12, further comprising regularly determining at least one third parameter resulting from a cardiac stimulation under the operating stimulation configuration and comparing it with the same parameter stored as reference parameter in the memory unit of the implantable medical device (102).

14. An arrangement for configuring an implantable medical device for stimulating a human or animal heart, the arrangement comprising:
an implantable medical device (102) for stimulating a human or animal heart;
a programming device (100) operatively coupled to the implantable medical device (102);
a plurality of electrode patches (108) that can be applied onto a body surface of a patient (105), wherein the plurality of electrode patches (108) is capable of sensing first signals being representative for an electric cardiac excitation propagation of a patient (105) when applied onto the body surface of the patient (105), wherein at least one of the plurality of electrode patches (108) comprises a sensor for determining a phrenic stimulation of the patient (105);
wherein the programming device (100) comprises a processor and a memory unit, wherein the memory unit comprises computer-readable code that causes the processor to execute a method comprising the following steps when executed on the processor:
a) stimulating (200), with the programming device (100) or the implantable medical device (102) operated under a test stimulation configuration, the heart (104) of a patient (105);
b) capturing (201), with the plurality of electrode patches (108) applied onto a body surface of the patient (105), first signals being representative for an electric cardiac excitation propagation of the patient (105) in response to the stimulation (200);
c) capturing (202), with at least one of the plurality of electrode patches (108) applied onto the body surface of the patient (105), a second signal being representative for an excitation of the diaphragm of the patient (105) and/or for a phrenic stimulation threshold;
d) determining (203), on the basis of the first signals at least one first parameter chosen from the group consisting of a cardiac stimulation threshold, a cardiac stimulation impedance, a cardiac stroke volume, a cardiac signal amplitude, an atrioventricular interval, and an interventricular interval;
e) determining (204), on the basis of the second signal, a phrenic stimulation threshold;
f) optionally repeating (205) steps a) to e);
g) setting (206), on the basis of the at least one first parameter and of the phrenic stimulation threshold, an operating stimulation configuration of the implantable medical device (102) that enables sufficient stimulation of the patient's heart (104) by the implantable medical device (102) and avoids at the same time a stimulation of the patient's diaphragm upon a cardiac stimulation by the implantable medical device (102).

15. The arrangement of claim 14, further comprising a remotely located home monitoring service center (113), wherein the programming device (100) is operatively coupled to the home monitoring service center (113) to be able to transfer data to the home monitoring service center (113).
